# EUROPEAN PATENT APPLICATION

(11) **EP 1 544 310 A2**
(43) Date of publication of application: **22.06.2005**
(21) Application number: 04257748.6
(22) Date of filing: 14.12.2004
(51) Int. Cl.: C12Q 1/68

(54) **Method and systems for characterizing a polymer**

(30) Priority: 19.12.2003 US 741119
(71) Applicant: Agilent Technologies, Inc., Palo Alto, CA 94306 (US)
(72) Inventor: Tom-Moy, May, San Carlos California 94070 (US); Pittaro, Richard J., San Carlos California 94070 (US); Myerholtz, Carl, Cupertino California 95014 (US)
(74) Representative: Tollett, Ian

(57) **Abstract**

Methods and systems for characterizing a polymer in a sample are provided. In the subject methods, a sample that includes a polymer labeled with at least one nanoparticle (40) is contacted with a nanopore (111, 350, 420) under conditions so that the polymer translocates through the nanopore (111, 350, 420). A signal is read from the nanopore to characterize the translocated polymer. The subject systems include a nanopore device (110) and a polymer that is labeled with at least one nanoparticle (40). Also provided is programming stored on a computer-readable medium for use in practicing the subject methods. Kits for use in practicing the subject methods are also provided.

SEQUENCE LISTING

<110>  Agilent Technologies, Inc.

<120>  Methods and Systems for Characterizing a Polymer

<130>  IT/KE/N15974

<150>  US 10/741,119
<151>  2003-12-19

<160>  8

<170>  PatentIn version 3.1

<210>  1
<211>  16
<212>  DNA
<213>  Artificial sequence

<220>
<223>  Synthetic DNA

<400>  1
atcgatcgat cgatcg                                                    16


<210>  2
<211>  37
<212>  DNA

<213>  Artificial sequence

<220>
<223>  Synthetic DNA

<400>  2
aatctaggga attcacagcg atgcgaattc gcaatta                             37


<210>  3
<211>  9
<212>  DNA
<213>  Artificial sequence

<220>
<223>  Synthetic DNA

<400>  3
aatctaggg                                                             9

<210>  4
<211>  16
<212>  DNA
<213>  Artificial sequence

<220>
<223>  Synthetic DNA

<400>  4
aattcacagc gatgcg                                                    16


<210>  5
<211>  12
<212>  DNA
<213>  Artificial sequence

<220>
<223>  Synthetic DNA

<400>  5
aattcgcaat ta                                                        12


<210>  6
<211>  12
<212>  DNA
<213>  Artificial sequence

<220>
<223>  Synthetic DNA

<400>  6
atcggttcgc at                                                        12


<210>  7
<211>  13
<212>  DNA
<213>  Artificial sequence

<220>
<223>  Synthetic DNA

<400>  7
tacgcgttga cga                                                       13


<210>  8
<211>  13

<212> DNA
<213> Artificial sequence

<220>
<223> Synthetic DNA

<400> 8
tacgcgttga cga 13

## Description

The field of this invention is polymer characterization, particularly nanoparticles for use as detectable labels in nanopore polymer characterization protocols.

Polymer characterization, including detection and sequencing, has recently been facilitated by the use of "nanopore" technology. This technology utilizes nanopores which are characterized by having dimensions on the order of nanometers. These nanopores may be naturally occurring pores in cell membranes (see, for example, U.S. Pat. No. 5,795,782) or artificially-produced nanopores such as solid state nanopores. Both types of nanopores have been used to "sense" or "read" the passage (i.e., translocation) of individual polymers through the nanopores, which passage yields information, e.g., in the form of electronic signals, about the passed polymer.

One way of sensing the passage of a polymer through the pore is to monitor ionic current through a solution filling the pore when a voltage is applied across the pore. Monitoring electron tunneling current and resonance electron tunneling current may also be employed for this purpose. For example, using ionic current the polymer of interest enters and passes through the pore, an electronic signature is generated (for example a reduction in a maximum current), and the electronic signature is detected, which indicates that the pore is partly or completely blocked by the polymer passing therethrough. These electronic signatures, which are characteristic of a particular polymer, may be used to distinguish molecules from each other and/or to distinguish the size of a polymer, e.g., distinguish different lengths of DNA.

There continues to be an interest in the development of new methods and systems to characterize polymers using nanopore technology. Of particular interest is the development of such methods and systems that employ unique labels that are easy to use and which provide unique electronic signals.

Methods and systems for characterizing a polymer in a sample are provided. In the subject methods, a sample that includes a polymer labeled with at least one nanoparticle is contacted with a nanopore under conditions so that the polymer translocates through the nanopore. A resultant signal is read from the nanopore to characterize the translocated polymer. The subject systems include a nanopore device and a polymer that is labeled with at least one nanoparticle. Also provided is programming stored on a computer-readable medium for use in practicing the subject methods. Kits for use in practicing the subject methods are also provided.

Preferred embodiments of the present invention are described below, by way of example only and with reference to the accompanying drawings, in which:
FIGS. 1A-1C show exemplary embodiments of a nucleic acid labeled with one or more nanoparticle labels according to a representative embodiment of the subject invention. Specifically, FIG. 1A shows an exemplary embodiment of a nucleic acid labeled with a single nanoparticle label, FIG. 1B shows an exemplary embodiment of a nucleic acid labeled with a plurality of the same type of nanoparticle labels and FIG. 1C shows an exemplary embodiment of a nucleic acid labeled with four different types of nanoparticle labels.
FIG. 2 shows an exemplary embodiment of a nucleic acid hybridized to a nanoparticle-labeled probe nucleic acid molecule according to a representative embodiment of the subject invention.
FIG. 3 shows an exemplary embodiment of a nucleic acid hybridized to a plurality of identical nanoparticle labeled oligonucleotides according to a representative embodiment of the subject invention.
FIG. 4 shows an exemplary embodiment of a nucleic acid hybridized to two different nanoparticle labeled oligonucleotides according to a representative embodiment of the subject invention.
FIG. 5 shows another exemplary embodiment of a nucleic acid hybridized to a plurality of distinct nanoparticle labeled oligonucleotides according to a representative embodiment of the subject invention.
FIG. 6A shows an exemplary embodiment of a nucleic acid labeled in accordance with the subject invention being passed through a pore of a nanopore device according to a representative embodiment of the subject invention and FIG. 6B shows the signal produced thereby.
FIGS. 7A and 7B show an exemplary embodiment of the subject invention employed to determine the size of a polymer of interest by labeling the ends of the polymer with nanoparticle labels according to a representative embodiment of the subject invention. Specifically, FIG. 7A shows the nanoparticle labeled polymers passing through a pore of a nanopore device and FIG. 7B shows the output signal produced thereby.
FIG. 8 shows an exemplary embodiment of the subject invention employed as an alternative to a restriction fragment polymorphism protocol to determine whether any polymorphisms are present in a nucleic acid according to a representative embodiment of the subject invention.
FIGS. 9A and 9B show an exemplary embodiment of the subject invention employed to sequence a nucleic acid of interest by labeling each nucleotide with a nanoparticle label according to a representative embodiment of the subject invention. Specifically, FIG. 9A shows the nanoparticle labeled nucleic acid passing through a pore of a nanopore device and FIG. 9B shows an exemplary output signal produced produced by such a passing.
FIGS. 10A and 10B show an exemplary embodiment of a nucleic acid strand labeled in accordance with the subject invention passing through a pore of a nanopore device (FIG. 10A) and the output signal produced thereby (FIG. 10B) according to a representative embodiment of the subject invention.
FIG. 11 shows an exemplary embodiment of the subject invention employed in an electron tunneling current protocol according to a representative embodiment of the subject invention.
FIGS. 12A and 12B show exemplary embodiments of unlabeled DNA passing through tunneling current electrodes and the output signal produced thereby (FIG. 12A) and nanoparticle labeled DNA passing through tunneling current electrodes and the output signal produced thereby (FIG. 12B) according to a representative embodiment of the subject invention.
FIGS. 13A and 13B shows an exemplary embodiment of nanoparticle labeled DNA passing through resonant tunneling current electrodes and the output signal produced thereby according to a representative embodiment of the subject invention.
FIG. 14 shows an exemplary embodiment of a magnetic detection circuit for detecting magnetic nanoparticle labels in accordance with the subject invention.

The term "polymer" refers to any compound that is made up of two or more monomeric units covalently bonded to each other, where the monomeric units may be the same or different, such that the polymer may be a homopolymer or a heteropolymer. Polymers include, but are not limited to, peptides and polypeptides, polysaccharides, nucleic acids carbohydrates, polyurethanes, polycarbonates, polyureas, polyethyleneimines, polyarylene sulfides, polysiloxanes, polyimides, polyacetates, polyamides, polyesters, polythioesters and the like, where the polymers may be naturally occurring or synthetic.

The term "nucleic acid" as used herein means a polymer composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in hybridization reactions, i.e., cooperative interactions through Pi electrons stacking and hydrogen bonds, such as Watson-Crick base pairing interactions, Wobble interactions, etc. Nucleic acids may be single or double stranded.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

The term "oligomer" is used herein to indicate a chemical entity that contains a plurality of monomers. As used herein, the terms "oligomer" and "polymer" are used interchangeably. Examples of oligomers and polymers include polydeoxyribonucleotides (DNA), polyribonucleotides (RNA), other polynucleotides which are C-glycosides of a purine or pyrimidine base, polypeptides (proteins), polysaccharides (starches, or polysugars), and other chemical entities that contain repeating units of like chemical structure.

The terms "nucleoside" and "nucleotide" are intended to include those moieties which contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

"Optional" or "optionally" means that the subsequently described circumstance may or may not occur, so that the description includes instances where the circumstance occurs and instances where it does not.

"Quantum dots" or "nanocrystals" herein used interchangeably are known in the art and are generally nanosized semiconductors that fluoresce (see, for example, Bruchez et al (1998) Semiconductor nanocrystals as fluorescent biological labels. Science 281, 2013-2016 and Chan, W. C. et al (1998) Quantum dot bioconjugates for ultrasensitive nonisotopic detection. Science 281, 2016-2018).

A "computer-based system" refers to the hardware means, software means, and data storage means used to perform certain functions and/or analyze the information of the present invention. The minimum hardware of the computer-based systems of the present invention may include a central processing unit (CPU), input means, output means, and data storage means. A skilled artisan can readily appreciate that any one of the currently available computer-based systems are suitable for use in the present invention. The data storage means may include any manufacture including a recording of information relating to the subject invention, or memory access means that can access such a manufacture.

To "record" data, programming or other information on a computer-readable medium refers to a process for storing information, using any such methods as are known in the art. Any convenient storage structure may be chosen, based on the means to access the stored information. A variety of data processor programs and formats may be used for data storage, e.g., word processing text file, databases format, etc.

A "processor" references any hardware and/or software combination that will perform the functions required of it. For example, a processor herein may be a programmable digital microprocessor such as available in the form of an electronic controller, mainframe, server or personal computer (desktop or portable). Suitable programming may be communicated from a remote location to the processor, or previously saved in a computer program product (such as a portable or fixed computer-readable storage medium, whether magnetic, optical or solid state device based). For example, a magnetic or optical disk may carry the programming, and can be read by a suitable disk reader communicating with a respective processor at its corresponding station.

Methods and systems for characterizing a polymer in a sample are provided. In the subject methods, a sample that includes a polymer labeled with at least one nanoparticle is contacted with a nanopore under conditions so that the polymer translocates through the nanopore. A signal is read from the nanopore to characterize the translocated polymer. The subject systems include a nanopore device and a polymer that is labeled with at least one nanoparticle to provide a nanoparticle label. Also provided is programming stored on a computer-readable medium for use in practicing the subject methods. Kits for use in practicing the subject methods are also provided.

Before the present invention is described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "an", and "the" include plural referents unless the context clearly dictates otherwise.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention.

The figures shown herein are not necessarily drawn to scale, with some components and features being exaggerated for clarity.

### OVERVIEW

As noted above, a feature of the subject invention is that the nanopore detected polymers, e.g., nanopore detected nucleic acids, are labeled with one or more nanoparticles. In general, the nanoparticle labeled polymers of the subject invention are capable of generating a distinctive, reproducible signal when passed or translocated through a nanopore. Translocation may be accomplished by employing an applied electric field, using atomic force tweezers, a magnetic force, and the like, i.e., any suitable method of moving a polymer through a nanopore. Signal generated from the nanoparticle labeled polymer upon passage through a nanopore is unique such that it is substantially different from a signal detected from the nanopore without a polymer therein or different from signal detected from the nanopore having the same polymer absent such a nanoparticle label translocated therethrough or different from the nanopore having the same polymer labeled with a different nanoparticle label. This detectable signal may be used in a variety of polymer characterization protocols, e.g., to determine the size of a polymer (e.g., a label positioned at the first and last positions of a polymer) and/or to identify each unit of a multi unit polymer and/or unit type and/or unit position within the polymer, etc. As such, the subject methods may be used in a variety of different applications.

In further describing the subject invention, the subject methods are described first, followed by a review of representative applications in which the subject methods find use, as well as a review of representative systems and kits that may be used in the practice of the subject methods.

### METHODS

As summarized above, the subject methods include labeling a polymer of interest with one or more nanoparticle labels to produce a nanoparticle labeled polymer. Once labeled, the polymer is passed or translocated through a nanopore providing distinctive, reproducible signals corresponding to each particular nanoparticle label attached to the polymer. Passage or translocation of the nanoparticle labeled polymer through the nanopore generates a detectable signal, which may be detected according to a variety of different protocols. The resultant detected signal is then employed to characterize the labeled polymer. As such, the subject methods can be viewed as including the following four substeps:
a) providing a nanoparticle labeled polymer;
b) translocating the nanoparticle labeled polymer through a nanopore;
c) detecting a signal generated by the translocation of the polymer; and
d) employing the detected signal to characterize the polymer.

Each of the above substeps is now described separately in greater detail below.

### Provision of Nanoparticle Labeled Polymer

As indicated above, generally the first step in the subject methods is to label a polymer of interest with one or more nanoparticles to produce a nanoparticle labeled polymer. Before elaborating further on various representative embodiments of producing a nanoparticle labeled polymer, representative types of polymers and nanoparticles that may be employed to label the same are reviewed first in greater detail.

### Representative Polymers

The term "polymer" as described above, refers to any compound that is made up of two or more units or monomeric units or subunits covalently bonded to each other, where the monomeric units may be the same or different, such that the polymer may be a homopolymer or a heteropolymer. Representative polymers include, but are not limited to, peptides and polypeptides, polysaccharides, nucleic acids (double and single stranded), carbohydrates, polyurethanes, polycarbonates, polyureas, polyethyleneimines, polyarylene sulfides, polysiloxanes, polyimides, polyacetates, polyamides, polyesters, polythioesters and the like, where the polymers may be naturally occurring or synthetic. The polymer to be characterized may be of any suitable size, with the only limitation being that it is able to translocate through a nanopore.

The subject invention is particularly well suited for characterizing a nucleic acid, e.g., the subject invention may be employed to identify and/or discriminate between sequences of a nucleic acid or nucleotides of a nucleic acid. The term "nucleic acid" as used herein means a polymer composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in hybridization reactions, i.e., cooperative interactions through Pi electrons stacking and hydrogen bonds, such as Watson-Crick base pairing interactions, Wobble interactions, etc. The subject invention may be used with any nucleic acid either naturally occurring or synthetic and may be single or double stranded.

While the subject nanoparticle labels are primarily described herein with respect to the characterization of nucleic acids, it will be readily apparent to those of skill in the art that a wide variety of polymers or constituents or analytes may be employed other than nucleic acids. Other constituents include both naturally occurring and synthetic constituents, e.g., biological analytes such as antibodies, receptors, ligands, proteins, viruses, bacteria, toxins, etc., and environmental constituents, e.g., toxins, pollutants, etc., and the like.

A polymer to be characterized in accordance with the subject invention may be naturally occurring or synthetic. For example, the sample that is screened in the subject invention, i.e., the sample containing or suspected of containing the polymer to be characterized, may be obtained from a variety of sources. Samples, as used herein, include, but are not limited to, biological fluids such as blood, cerebrospinal fluid, tears, saliva, lymph, dialysis fluid, semen and the like; organ or tissue culture derived fluids; food and fluids extracted from cells or physiological tissues, where the cells may be dissociated, in the case of solid tissues, or tissue sections may be analyzed. In some embodiments, a lysate of the cells may be prepared. Other samples of interest include environmental samples, such as plant tissue samples, ground water samples, soil samples, and derivatives of such samples.

### Nanoparticle Labels

A number of different types of nanoparticle labels, each having a distinctive, reproducible signal are provided by the subject invention and thus a wide variety of polymer labeling schemes may be employed. For example, a polymer may be labeled with more than one nanoparticle label where the various nanoparticle labels employed may provide the same or different signals. For example, in certain embodiments two or more different types of nanoparticle labels may be employed to label a given polymer. By different type of nanoparticle label it is meant that the different types of nanoparticle labels provide different or rather distinct, detectable signals upon detection so that the respective signals may be attributed to the respective nanoparticles. To this end, different portions or different units of a polymer may be labeled with a different type of nanoparticle label. As such, each portion or each labeled unit may be differentially detected as it is passed through a nanopore by observing, for example ionic, tunneling and resonance tunneling current. Accordingly, nanoparticle labels may be considered to be of different types if they provide different, distinct, detectable signals during translocation through a nanopore such that their signals are distinguishable from each other.

A feature of the subject invention is that the distinctive signal generated by each different type of nanoparticle label is a function of at least one of one or more physical and/or chemical properties of that type of nanoparticle label, e.g., a function of at least one of (1) the material of the nanoparticle label, and/or (2) the size of the nanoparticle label. The materials of interest in the subject invention include those that are conductive, semi-conductive, magnetic (including superparamagnetic), both conductive (or semi-conductive) and magnetic. The sizes of the nanoparticle labels fall within a wide range, thus further increasing the number of different, distinguishable labels of the subject invention. Generally, the subject nanoparticle labels are sized to be stably associated with a polymer and translocated through a nanopore. Accordingly, the nanoparticle labels of the subject invention are of nanometer dimensions.

As described above, in certain embodiments the detectable change in signal is due at least in part to the material of a subject nanoparticle label. Accordingly, the subject nanoparticle labels include a material that is able to elicit or produce a detectable change in the current, e.g., the ionic current and/or tunneling and/or resonant tunneling current and/or a change in a magnetic field or magnetic circuit, during translocations (e.g., voltage-driven translocations or the like) of the nanoparticle label through a nanopore. Accordingly, the nanoparticle labels of the subject invention include, i.e., are fabricated in whole or in part from, any suitable material that is capable of eliciting a detectable change in the current flow, whether ionic, electron, or the like, or a detectable change in a magnetic circuit through a nanopore. Such materials may generally be characterized as conductors, semiconductors, magnetic materials- including materials that posses more than one of these properties. Accordingly, any material (or combination of materials) that is conductive, semiconductive, magnetic, both conductive (or semiconductive) and magnetic, may be employed as a nanoparticle label in the subject invention, e.g., any material that is capable of acting like a conductor or a metal or semiconductor in regards to conductivity may be employed, as may be any material that is capable of acting like a magnetic particle in regards to, e.g., modifying magnetic susceptibility, e.g., such as a ferromagnetic nanoparticle label and the like. Accordingly, in many embodiments the nanoparticle labels are highly conductive particles, e.g., high conductivity metals, where by "high conductivity" is meant that the resistivity is less than about 10 x 10⁻⁸ Ohm-Meters.

Representative nanoparticle label materials include, but are not limited to, metals and metal alloys and oxides thereof, e.g., gold, silver, copper, tin, titanium, iron, cobalt, chromium, molybdeneum, vanadium, aluminum, zinc, bismuth, zirconia, tungsten carbide, magnesium, cerium, nanoparticle -polymer hybrids, and the like, alloys thereof and oxides thereof (e.g., copper oxide, tin oxide, titanium dioxide, indium tin oxide, antimony tin oxide, barium titanate, calcium oxide), where a given nanoparticle label may be fabricated from a combination of such materials such that a given nanoparticle label may be fabricated from two or more materials. In certain embodiments the nanoparticle label is a nanocrystal or quantum dot.

The nanoparticle labels of the subject invention may be a composite, a laminate, etc. A "composite" is a composition made of different materials. A nanoparticle composite may be a block composite, e.g., an A-B block composite, an A-B-A block composite, an A-B-C block composite, and the like, where the number of different materials that may be employed may vary depending on the particular nanoparticle. The composite may be heterogeneous, i.e., in which the materials are distinct or in separate phases, or a homogeneous combination of unlike materials. As used herein, the term "composite" is used to include a "laminate" composite. A "laminate" refers to a composite material formed from several different bonded layers of same or different materials. In certain embodiments, a nanoparticle label may be fabricated from gold and silver, e.g., gold coated or layered with silver or *vice versa.* Nanoparticles that may be employed in the subject invention include, but are not limited to, nanoparticles such as Nanotek® Copper Oxide, Nanotek® Indium Tin Oxide, Nanotek® Titanium Oxide from Nanophase Technologies Corporation in Illinois and nanoparticles, such as Nanogold® , from Nanoprobes, Inc., in New York. Commercially available magnetic nanoparticles may be employed in certain embodiments, e.g., available from Reade Advanced Materials, Seradyn, Inc., and Bangs Laboratories, Inc.

As noted above, one or more nanoparticle labels may be in the form of semiconductor which may be in the form of a quantum dot. In certain embodiments, a given nanoparticle label may include a particle, e.g., a latex bead or the like, filled with a plurality of quantum dots. Thus, by combining a variety of quantum dots in a single particle and varying the number and/or type of quantum dots employed, a variety of different, distinguishable nanoparticle labels may be provided.

In many embodiments, a plurality of the subject nanoparticle labels are stably associated with a given polymer (e.g., different portions of the polymer or different units that make up the polymer), where some or all of the nanoparticle labels may be of different materials (and/or sizes). In such instances, because these nanoparticle labels differ at least in regards to material, each interferes with the current or magnetic field or circuit in a unique way allowing it to be singled-out or distinguished from the other nanoparticle labels that are of different materials (and/or sizes) during detection. Accordingly, the nanoparticle labels associated with a polymer or one or more monomers may be distinctly detected in their order of appearance through a nanopore. In this regard, the signals obtained from the different materials of the nanoparticle labels as they are translocated through a nanopore differ due at least in part to the different materials of the nanoparticle labels, e.g., by modifying the electron current flow within the nanopore with a conductive or semiconductive nanoparticle, e.g., in tunneling and resonant tunneling protocols. For example, in certain embodiments at least two nanoparticles stably associated with a given polymer may be of different materials such that each interferes with the current or magnetic field in a unique way due at least in part to the particular material, e.g., different conductivity properties (e.g., one or more nanoparticles may be gold and one or more may be silver), where both are bound to the polymer of interest, e.g., at different positions or locations of the polymer. For example, one nanoparticle label may be positioned at one end of a polymer such as at a first end of a single or double strand length of DNA and the other nanoparticle label at the other or second end of the polymer. Accordingly, the length of the DNA may be determined based on the detection of the nanoparticle labels, e.g., based on the time differential between detection of the nanoparticle labels. An analogous protocol may be employed for other types of detection. For example, in using magnetic particles, one magnetic nanoparticle label having a first material composition (and/or first nanoparticle size) may be positioned at one end of a polymer such as at a first end of a single or double strand length of DNA and a second magnetic nanoparticle label having the same or having a second material composition (and/or second nanoparticle size) at the other or second end of the polymer. Accordingly, the length of the polymer such as DNA may be determined based on the detection of the nanoparticle labels, e.g., based on the time differential between detection of the nanoparticle labels.

As noted above, the nanoparticle labels of the subject invention may also be electronically distinguishable due to their size (and/or material), where the size of a given nanoparticle label causes a detectable partial or complete blockage of current flow through the nanopore. As the subject labels are nanoparticles, they have nanometer dimensions. Specifically, while the size of a given nanoparticle is large enough to produce a detectable effect in the current flow or magnetic susceptibility through a nanopore, the size is small enough to be able to translocate through the nanopore from one side of the nanopore to another. In other words, each of the nanoparticle labels are large enough to elicit a distinctive, reproducible signal and yet small enough that they are of nanometer proportions.

For example, the size of a nanoparticle label according to the subject invention is usually small enough to fit through a nanopore (either naturally or synthetically produced) having a diameter that ranges from about 2 nm to about 35 nm, e.g., about 0.5 nm to about 1 nm for a nanopore having a size that is able to accommodate, e.g., amino acids, sugars and nucleotides. Thus, the only limitation with respect to the physical dimensions of a nanoparticle label is that it be able to pass or translocate through a nanopore from a first side to a second side and also that it be able to elicit a distinctive, reproducible signal or current signature (where the signal may be amplified in certain embodiments) such as a blockade signal or blockade signature or signal related to a change in magnetic susceptibility, etc. Accordingly, the nanoparticles have a size that is at least somewhat smaller than the diameter of the nanopore through which it is passed. As such, the size of a nanoparticle according to the subject invention may range from about 0.5 nm to about 35.0 nm, e.g., from about 0.8 nm to about 30 nm, e.g., from about 0.5 nm to about 15.0 nm, e.g., from about 0.8 nm to about 5.0 nm.

In many embodiments, a plurality of the subject nanoparticle labels are stably associated with a given polymer (e.g., different portions of the polymer or different units that make up the polymer) where some or all of the nanoparticle labels may be of different sizes (and/or materials). In such instances, because these nanoparticle labels differ at least in regards to size, each interferes with the current or magnetic field or circuit in a unique way allowing it to be singled out or distinguished from the other nanoparticle labels. Accordingly, the nanoparticle labels associated with a polymer or one or more monomers may be detected in their order of appearance through a nanopore. In this regard, the signals obtained from the different sizes of nanoparticles differ due at least in part to the size differential of the nanoparticle labels. For example, in certain embodiments at least two nanoparticle labels may be of different sizes (and/or materials). For example, in certain embodiments at least two nanoparticle labels stably associated with a given polymer may be of different sizes where the sizes differ by an amount that is at least great enough to provide a distinguishable or unique change in current such that each interferes with the current in a unique way due at least in part to size. For example, in certain embodiments at least two nanoparticle labels stably associated with a given polymer are of different sizes, e.g., one or more nanoparticles may be about 0.8 nm and one or more may be about 1.4 nm, where both are bound to the polymer of interest, e.g., at different positions or locations of the polymer. Particular sizes of magnetic nanoparticle labels (and/or material compositions) or ranges thereof may be accomplished by various techniques such as employing one or more surfactants such as a combination of oleic acid/oleyl amine to control the nanoparticle growth during fabrication of the nanoparticle and/or by varying metal/surfactant ratio or synthesis temperature during fabrication of a nanoparticle (see, for example, Baselt, et al., Biosens. Bioelectron 13, 731 (1998)).

As will be apparent, a number of distinctive nanoparticle labels, i.e., different nanoparticle types, may be provided by exploiting one or more physical or chemical properties of the nanoparticle labels. For example, in certain embodiments signal obtained from each nanoparticle label employed in a given protocol may be distinguishable based solely on the sizes, e.g., the differential in sizes, of the nanoparticle labels employed while in certain other embodiments signal obtained from each nanoparticle label employed in a given protocol may be distinguishable based solely on the material, e.g., the differential in the materials, of the nanoparticle labels employed. Signal differentiation may also be accomplished by exploiting two or more properties of the nanoparticle labels. For example, both nanoparticle size and nanoparticle material properties may be exploited to interfere with the current in a unique way such that each nanoparticle label provides a distinct, distinguishable signal. In other words, by employing different sized nanoparticle labels with nanoparticle labels of different sizes, e.g., different sized nanoparticle labels made of different materials, a variety of detectably distinguishable nanoparticle labels may be provided.

Still further, a variety of nanoparticle labels may be provided by combining different materials with different sizes in a given protocol so as to increase the number of different distinguishable nanoparticles that may be employed in a given protocol, each having unique properties such that the current signature obtained from a given nanoparticle label is distinct to that nanoparticle label and is based on the material and size properties of the nanoparticle label and thus distinguishable from the signal generated from any other nanoparticle label. For example, by just permuting two different materials, e.g., gold and silver, with two different sizes, e.g., 0.8 nm and 1.4 nm, four different nanoparticle labels may be provided (gold/0.8 nm, gold/1.4nm, silver/0.8 nm and silver/1.4nm) where each nanoparticle label will elicit a reproducible, distinguishable signal as it translocates through a nanopore. This labeling scheme may be used, for example, to differentiate between specific sequences of oligonucleotides or to differentiate between different units or monomers of a polymer such that each different unit of a polymer may be labeled with a different, distinguishable nanoparticle label. Such a labeling scheme may be employed to label the four different nucleotide types of a nucleic acid such as DNA, e.g., by stably associating each different nucleotide type with a specific nanoparticle label to provide differentiation between the nucleotides, e.g., for sequencing or other characterization analysis. As a number of different materials and sizes may be employed in the fabrication of the subject nanoparticle labels in accordance with the subject invention as described above, it will be apparent that a large number of different, distinguishable nanoparticle labels may be employed such that each interferes with the current in a unique way.

### Nanoparticle Labeling of Polymers

To produce nanoparticle labeled polymers, each nanoparticle label is stably bound to a polymer of interest, e.g., bound to a particular nucleic acid sequence or particular unit or monomeric residue of a polymer (e.g., a particular nucleotide type). The nanoparticle labels may be directly or indirectly associated with the polymer of interest, depending at least in part on the nature of the polymer to be characterized and the particular application in which the subject methods are being employed. In direct labeling approaches, one or more nanoparticles are directly bound to one or more residues of the polymer. In indirect labeling approaches, one or more nanoparticles are stably associated with a polymer by having the nanoparticles stably associated with a polymer binding moiety that specifically binds to a portion or some or all of the units of the polymer, e.g., one or more specific nucleotide bases or residues, e.g., a defined region, of a nucleic acid.

In direct labeling protocols as summarized above, one or more residues of the polymer are bound to a nanoparticle label. A particular monomeric residue of a polymer may be attached either directly or indirectly to a given nanoparticle label. For example, a nanoparticle may be directly bound, either covalently or non-covalently, to a monomeric residue of the polymer. Alternatively, a linking group may be used for binding a nanoparticle label to a monomeric residue of the polymer. Certain linking groups may react with specific moieties of the polymer of interest, e.g., with thiol, primary amino and carbohydrate groups, etc. Commercially available linkers of this sort include, but are not limited to monomaleimido-undecagold, mono-sulfo-NHS-undecagold and mon-amino undecagold available from Nanoprobes, Inc. The linking group may be a variety of different moieties, where such linking groups include, but are not limited to disulfide groups, restriction sites, photocleavable groups, avidin-biotin conjugates, strepavidin-biotin conjugates, and the like.

In a representative embodiment, guanine residues of a nucleic acid may be labeled with a nanoparticle label as follows. In this representative embodiment, the Universal Linking System ("ULS"), e.g., Biotin-Chem-Link available from Roche, is employed to specifically functionalize the guanine residues with biotin. In this step, to be functionalized nucleic acids are contacted with Biotin-Chem-Link and nuclease-free water under conditions suitable to provide for the covalent binding of the biotin to guanine residues of the nucleic acid. The incubation time for this protocol should be sufficient for the biotin to bind available guanine residues. Generally, from about 30 minutes to about 1 hr is sufficient, usually 30 minutes sufficing. Incubation is generally performed at temperatures that range from about 75 °C to about 90 °C, usually about 85 °C. After incubation, the reaction is stopped with a suitable stop reagent. The biotin-conjugated probes are then contacted with strepavidin-conjugated nanoparticle labels under conditions sufficient for the biotin-conjugated probes to bind available strepavidin-conjugated nanoparticle labels. Generally, from about 30 minutes to about 1 hr is sufficient, usually 30 minutes sufficing. Incubation is generally performed at room temperature. In certain embodiments, the nanoparticle label may be enhanced further, e.g., by using the nanoparticle label as a "seed" for the development of another material, e.g., silver, about the nanoparticle label seed to easily transform the starting nanoparticle label from a first material having a first size to a second material having a second size thus providing distinct electronic signatures between the first, original nanoparticle label and the second, transformed nanoparticle label and due to the differences in size and/or material.

Where one wishes to bind a nanoparticle label to non-guanine residues of a nucleic acid polymer, analogous approaches are known and may be employed (see for example "Protocols for Oligonucleotide Conjugates", Methods in Molecular Biology, Vol. 26, Humana Press, Totowa, New Jersey (1994). For example, for nanoparticle attachment to nucleotide (adenine, cytosine, guanine and thymine/uracil) residues, one may modify nucleobases, e.g., to provide attached side chains which may bind a nanoparticle label, e.g., using a binding agent or linker. These modified nucleobases may be used in the synthesis of a oligonucleotide, e.g., which may be used as a nanoparticle labeled probe. A variety of positions or sites of a base (indicated below by arrows) may be employed for modifying a nucleic acid base, as shown below: 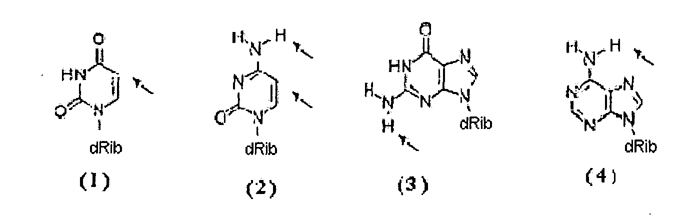 where base no. 1 shows a position that may be modified for uracil (thymine), base no. 2 shows positions that may be modified for cytosine, base no. 3 shows a position that may be modified guanine, and base no. 4 shows a position that may be modified for adenine.

The above-described nucleic acid bases show positions that may be modified for, e.g., introducing side arms into nucleic acid bases which side arms may then be employed in the attachment of a nanoparticle label such as by employing suitable linker (e.g., a biotin-strepavidin or the like). For example, in many embodiments the produced side arms include an attached amino group which enables attachment of, e.g., biotin conjugates.

For example, modified bases that may be employed include the following: 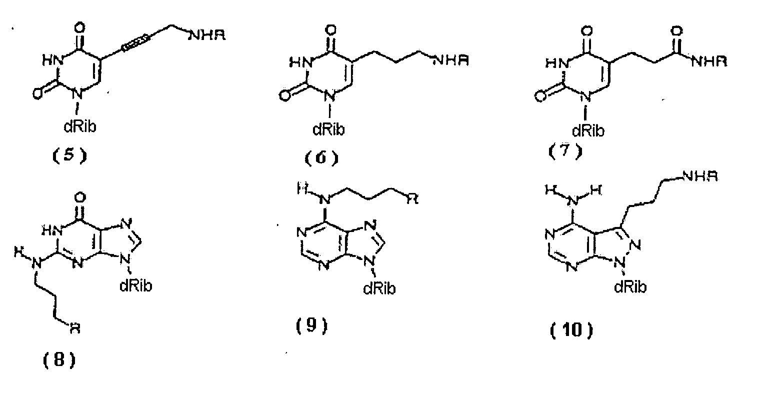 where base no. 5 is 5-(3-aminopropynyl)-2'-deoxyuridine, base no. 6 is 3 aminopropyl 2' deoxyuridine, base no. 7 is 5-(carbamoylethyl)-2'-deoxyuridine, base no. 8 is 3 deaza 3 substituted 2'-deoxyguanidine, base no. 9 is a deoxyadenosine base, and base no. 10 is pyrazole [3,4-d] deoxyadeosine.

Accordingly, a suitable nanoparticle may then be stably associated with a modified base and, either prior or subsequent to nanoparticle association, the modified bases may be incorporated into a synthesized oligonucleotide.

To stably associate a nanoparticle label with a modified base, a variety of linking groups may be employed, e.g., biotin conjugates. In certain embodiments, to attach a nanoparticle label to a modified base, e.g., used to synthesis a oligonucleotide, a suitable linking molecule such as biotin may be contacted with a given side arm of the modified oligonucleotide base under conditions suitable to stably associate or attach the linking group such as biotin to the base. A linking group labeled nanoparticle (e.g., strepavidin labeled gold or silver nanoparticles), for example, may then be contacted with the modified oligonucleotides under suitable conditions to attach the nanoparticle to the corresponding linker (e.g., biotin) of the base.

Where a given polymer is indirectly labeled with one or more nanoparticle labels, the polymer is contacted under suitable conditions with a nanoparticle labeled binding agent that specifically binds to the polymer. In these embodiments, the binding agent may be a variety of different moieties, depending on the nature of the polymer to be indirectly labeled, where representative binding agents include, but are not limited to: a ligand or receptor, proteins, including antibodies and binding fragments thereof, and nucleic acids, e.g., oligonucleotides, including deoxyribo-oligonucleotides and ribo-oligonucleotides. The nanoparticle labeled binding agent may be prepared using any convenient protocol, including the direct labeling protocols discussed above.

Methodology known in the art may be employed for chemically modifying and derivitizing a nanoparticle, see for example "Synthesis, Functionalization and Surface Treatment of Nanoparticles" edited by Marie-Isabelle Baraton, American Scientific Publishers, Los Angeles (2002) and "Surface Modification of Functional Nanoparticles Controlled Drug Delivery", J. of Dispersion Science and Technology Vol. 24(2003). Functionalized nanoparticles that may be employed in the subject invention include carbohydrate or PEG functionalized nanoparticles, peptide functionalized nanoparticles (e.g., from Alnis Biosciences, Inc.), carboxy functionalized magnetic nanoparticles (e.g., from Bang Laboratories, Inc. and Seradyn, Inc.), and the like. For example, a 3-thiol or 5-thiol linker may be employed to attach a nanoparticle (e.g., gold nanoparticle, silver nanoparticle, etc.) to an end of a nucleic acid. (see, e.g., Chad Mirkin et al. Storhoff et. al, J. Am. Chem. Soc 120, 1959 (1998)). In certain embodiments, nanoparticles, e.g., gold nanoparticles, may be functionalized with alkanethiols using ligand exchange reaction of phosphine-stabilized nanoparticle precursors with ω-functionalized alkanethiols, e.g., to prepare ω-functionalized nanoparticles with diameters of 0.8 nm to 1.5 nm (see, e.g., Gerd H. Woehrle, et al. "Improved Synthesis of Small (dCORE = 1.5 nm) Phosphine-stabilized Nanoparticles" J. Am. Chem. Soc., 2000, 122, 12890-12891). In certain embodiments, nanoparticle, e.g., a gold nanoparticle, may be provided with carboxy functional groups by contacting the nanoparticle with an organosilane. Thereafter, following activation of the nanoparticle with carbodiimide a suitable moiety such as strepavidin or the like may be stably attached to the nanoparticle via the provided functionalization. Methodology for functionalizing magnetic nanoparticles may also be found, e.g., in Catherine C. Berry and Adam S.G. Curtis, Functionalisation of magnetic nanoparticles for applications in biomedicine, J. Phys. D: Appl. Phys. 36 (2003) R198-R206.

Following preparation of a nanoparticle labeled binding agent, the polymer, e.g., nucleic acid of interest, is then contacted with the nanoparticle labeled binding agent under conditions sufficient for specific binding to occur to produce the desired nanoparticle labeled polymer. In general, following contact of one or more nanoparticle labeled binding agents with the polymer of interest, the resultant reaction mixture is incubated for a sufficient period time for the nanoparticle labeled binding agents to bind to the available polymers of interest, e.g., for any specific binding interactions, e.g., ligand-receptor binding, hybridization, etc., between a nanoparticle label and polymer or specific portion of the polymer, e.g., a particular sequence of a nucleic acid (if present), to occur. The particular incubation conditions will vary depending on the specifics of the polymer of interest and the nanoparticle label, where such conditions can be readily determined by those of skill in the art. For example, where the polymer of interest is nucleic acid such as DNA and the specific binding agent is a nanoparticle labeled probe having a sequence complementary to the sequence of the DNA of interest, conditions sufficient for hybridization of the nanoparticle labeled probe and the DNA of interest are employed, where the conditions will generally, though not always, be stringent conditions, for example, at 50°C or higher and 0.1×SSC (15 mM sodium chloride/01.5 mM sodium citrate). In this manner, the nanoparticle labeled probe is hybridized to its complementary sequence of the nucleic acid of interest (i.e., hybridized to a "target" nucleic acid) to provide a nanoparticle labeled target nucleic acid.

Regardless of whether a direct or indirect nanoparticle labeling scheme is employed, in certain embodiments following complex formation between nanoparticle labels and the polymer of interest, any unbound or free nanoparticle labels are separated from the population of nanoparticle labeled polymers. Any convenient separation protocol may be employed, where the particular separation protocol that is chosen in a given assay will depend, e.g., on the nature of the polymer. For example, centrifugation protocols, methods that discriminate based on size, etc. may be employed. Once the unbound nanoparticle labels are separated from the nanoparticle label/polymer complexes, the nanoparticle label/polymer complexes may then be translocated through a nanopore for characterization.

As indicated above, a variety of nanoparticle labeling schemes may be employed ranging from using a single nanoparticle label for a given polymer to a plurality of nanoparticle labels for a given polymer, where some or all of the nanoparticle labels employed may be different types of nanoparticle labels such that each different type of nanoparticle labels provides a unique, characteristic detectable signal during translocation. For example, a single nanoparticle label may be employed or a plurality of the same type or different type (or a mix of the same type and different type) of nanoparticle labels may be employed at various positions of the polymer. Accordingly, a given polymer, e.g., a nucleic acid molecule, may be labeled in a number of locations along the molecule's length. Because the individual sub-units of the polymer, e.g., nucleotides in the case of a nucleic acid, interact with the nanopore detector in sequential order, information regarding the location and composition of a plurality of labeled sites along a single molecule can be obtained using the subject invention.

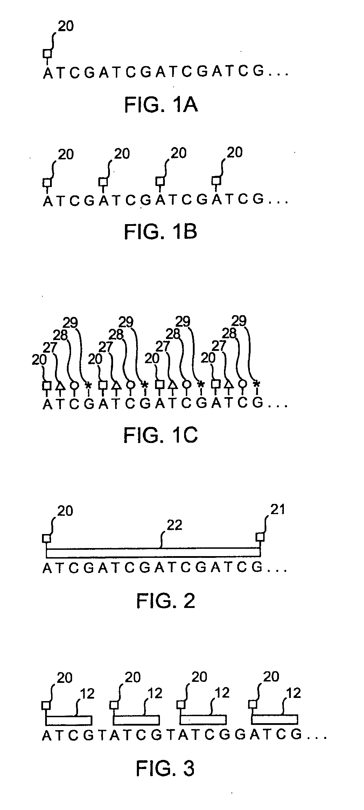

FIGS. 1-5 illustrate exemplary embodiments of various labeling schemes for a given nucleic acid, where such labeling schemes are exemplary only and are in no way intended to limit the scope of the invention. As noted above, the nanoparticle labels may be directly bound to specific nucleotide residues (A, C, T or G) or indirectly bound by way of a binding agent, e.g., complementary oligonucleotide or the like, to specific sequences of a nucleic acid. FIG. 1A shows a nucleic acid (ATCGATCGATCGATCG (SEQ ID NO:01)) having only a first nanoparticle label 20 represented by a square and which is bound directly to a residue at a first end (e.g., 5' or 3') of the nucleic acid (of course the nanoparticle label need not be positioned on an end). FIG. 1B shows the nucleic acid of FIG. 1A labeled with a plurality of nanoparticle labels to a specific nucleotide. In the embodiment of FIG. 1B, all A bases are labeled with the same nanoparticle label 20, but it will be apparent that one or more of the other bases (C, T or G) may be analogously labeled with different nanoparticle labels such as shown in the embodiment of FIG. 1C wherein each type of nucleotide is labeled with a different nanoparticle label (represented by nanoparticle labels 20, 27, 28 and 29) and signals provided from the different nanoparticle labels are detectably distinguishable from each other due to the materials and/or sizes of the labels.

FIG. 2 shows nanoparticle labels 20 and 21 bound to both ends of a known sequence probe 22 (the binding agent) to label the polymer sequence ATCGATCGATCGATCG (SEQ ID NO:01), where the labeled sequence may correspond to the entire length of a particular nucleic acid molecule or may be a portion thereof. While the nanoparticle labels are shown as different (represented as an open square 20 and closed square 21) such that the signal provided from the different nanoparticle labels are detectably distinguishable from each other due to the materials and/or sizes of the labels, the nanoparticle labels may be the same in certain embodiments. Furthermore, it will be apparent that a number of nanoparticle labels may be employed with a given binding agent which may be the same or different, depending on the size of the binding agent, etc.

FIG. 3 shows another exemplary embodiment of a labeling scheme wherein a plurality of first nanoparticle labeled binding agents 12 having nanoparticle label 20 are employed to label all of regions of a nucleic acid having the specific sequence ATCG.

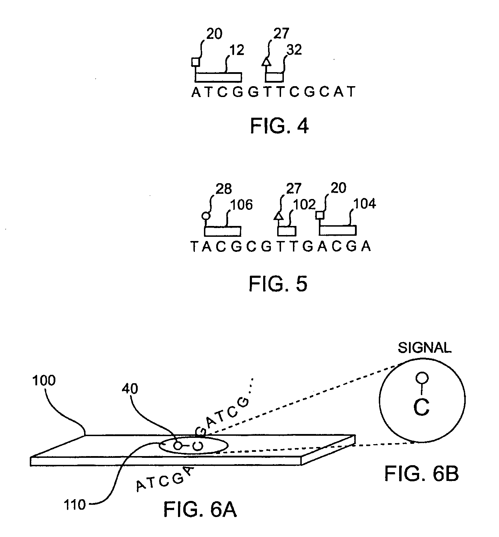

FIG. 4 shows another exemplary embodiment of a labeling scheme for labeling a polymer (SEQ ID NO:06). This labeling scheme employs a second nanoparticle label 32 having a second nanoparticle label 27 represented, where signal provided from the first and second labels, 20 and 27 respectively, are detectably distinguishable from each other due to the materials and/or sizes of the labels. For example, the two different nanoparticle labels 20 and 27 may differ in material and/or size such that the nanoparticle labels may be of the same material but differ in size (e.g., 0.8 nm and 1.4 nm), or may be of different material (e.g., gold and silver), but have the same size or may be of different materials and different sizes.

FIG. 5 shows yet another labeling scheme for labeling a polymer (SEQ ID NO:07). This labeling scheme employs three different nanoparticle labeled probes 102, 104 and 106, each labeled with a different type of nanoparticle label 27, 20 and 28 respectively, are employed to label a given polymer such that signal provided from the different nanoparticle labels are detectably distinguishable from each other due to the materials and/or sizes of the labels. Such a labeling scheme may be employed, e.g., to sequence a nucleic acid.

### Nanoparticle Labeled Polymer Translocation

Regardless of the particular labeling scheme employed, once labeled the nanoparticle labeled polymer is translocated through a nanopore. In many embodiments the nanoparticle labeled polymer is translocated during this step under an applied electric field, using atomic force tweezers, using a magnetic force, and the like.

The nanopore device that is employed in the translocation step may be a device that includes a nanopore inserted into a thin film with means for applying an electric field across the nanopore and for measuring the resultant signal at the nanopore or may be a solid state device, e.g., nanopores in materials such as a silicon-based chip (e.g., Si₃N₄) fabricated using ion beam sculpting and having nanoelectrodes positioned adjacent the pore (see for example Li, J., D. Stein, C. McMullan, D. Branston, M.J. Aziz, and J.A. Golovchenko; Ion Beam Sculpting at Nanometre Length Scales. Nature 412: 166-169 (2001). Representative nanopore devices are also disclosed in U.S. Patent Nos. 6,465,193; 6,428,959; 6,267,872 and 6,015,874; the disclosures of which are herein incorporated by reference. By "nanopore" is meant a structure having a channel or pore with a diameter of "nano" dimensions, where the inner diameter of the pore or channel typically ranges from about 1 to 10, usually from about 1 to 5 and more usually from about 1 to 2 nm. The nanopore may be synthetic or naturally occurring, where naturally occurring nanopores include oligomeric protein channels, such as porins, gramicidins, and synthetic peptides and the like, where a particularly preferred protein channel is the self-assembled heptameric channel of α -hemolysin. In one embodiment, the thin film into which the nanopore is inserted is a lipid bilayer fabricated from a wide variety of one or more different lipids, where suitable lipids include: phosphatidlycholine, phosphatidylserine, phosphatidylethanolamine, glycerol mono-oleate, and cholesterol. A variety of suitable thin film support devices have been reported in the literature that may be used to support a nanopore used to detect the subject nanoparticle labels. Such devices include those described in: Brutyan et al., Biochimica et Biophysica Acta (1995) 1236:339-344; Wonderlin et al., Biophys. J. (1990) 58:289-297; Suarez-Isla et al. Biochemistry (1983) 22:2319-2323; as well as in U.S. Patent No. 6,465,193; 6,428,959; 6,267,872 and 6,015,874; the disclosures of which are herein incorporated by reference.

In translocating the nanoparticle labeled polymer through the nanopore, the first step is to place a labeled nanoparticle label/polymer complex, on a first side of a suitable nanopore. The nanoparticle label/polymer complex may be in an aqueous solution, e.g., a buffered solution, where the solution may include one or more dissolved salts (e.g., in protocols measuring ionic current ), such as potassium chloride and the like, and the pH ranges from about 6.0 to 9.0, and more usually from about 7.0 to 8.5. The solution on a first side of the nanopore may be the same or different from the solution on the second side and may also be an ionic buffered solution. In tunneling current protocols, the polymer may be in air, methanol, propanol or butanol, water, or in suitable buffer such as a suitable buffer such as one that includes 1.0 M KCl, 10 mM Tris-Cl pH 8.5, 1mM EDTA and 0.05% Triton XL-80N or variations of such a buffer, e.g., nanopore buffer and methanol (e.g., 20% methanol) or the nanopore buffer with higher percentages of surfactant, e.g., 1% or 2% Triton XL-80N). Suitable solvents may also be employed, e.g., used with a nanopore buffer or other solution, such as polyethylene glycol. After the labeled nanoparticle label/polymer complex is placed on the first side of the nanopore, the labeled nanoparticle label/polymer complex is moved or translocated through the nanopore and the signal provided by the labeled nanoparticle label/polymer complex in the nanopore is detected.

For example, in certain embodiments an electric field is applied across the pore using electrodes positioned in the first and second side of the pore. The electric field that may be applied is sufficient to move or translocate the labeled nanoparticle label/polymer complex through the nanopore, where field strengths that range from about 10⁵ volts per centimeter to about 10⁶ volts per cm may be employed to cause a polymer such as a nucleic acid to translocate the nanopore. Field strengths up to about 10⁹ Volts per cm may be employed for tunneling and resonant tunneling measurements. In magnetic nanoparticle detection, a change of magnetic susceptibility (or reluctance) or the like through the nanopore, caused by the magnetic nanoparticle label, may be detected. For example, in certain embodiments a magnetic circuit may be provided about the nanopore, and the passage of a subject magnetic nanoparticle label interacts with the circuit in a manner to provide an observable change in magnetic susceptibility.

### Nanopore Signal Detection

During translocation of a subject nanoparticle label through a nanopore, a reproducible, characteristic signal, such as a reproducible, characteristic electronic and/or magnetic signal, may be detected due at least in part to the particular size and/or material of a given nanoparticle label. For example, in certain embodiments a distinct current signature or current profile may be obtained based on a particular nanoparticle label. For example, the passage of a labeled polymer such as an individual nanoparticle labeled nucleic acid strand through a nanopore may be observed as a transient decrease or spike in current, e.g., in ionic current, and/or the current blockage caused by a labeled-polymer translocation may be observed. In regards to detecting magnetic nanoparticle labels, such may be accomplished by using a spin valve sensor or giant magnetoresistive ("GMR") head.

Due to the particular characteristics and properties of a subject nanoparticle label(s) employed with the polymer of interest, when translocated through a nanopore, these nanoparticle labels are able to cause a detectable change in the electronic signature and thus are easily and readily detectable. In many embodiments, during translocation of the labeled nanoparticle label/polymer complex through the nanopore, the ion current through the pore is measured. Measurement rates may vary, e.g., measurement rates may range from about a few kHz (e.g., for a sizing application ) to tens of MHz (e.g., for a sequencing application).

While the subject invention is described primarily with respect to reading signals representative of ionic current, it is to be understood that such is for ease of description only and is not intended to limit the scope of the invention. It will be apparent to one of skill in the art that the subject invention is applicable for detecting a variety of signals such as those representative of ionic current flow, electron tunneling current flow, electron resonance tunneling current flow, changes in a magnetic field or circuit, variations in ionic and tunneling current flow, etc., such that in at least some embodiments, a current tunneling that is characteristic of the labeled polymer of interest is detected. For example, certain embodiments include employing an appropriate voltage bias to drive a polymer or the units or monomers of a polymer, such as DNA, to move in strictly single-file order through a nanopore's very small volume of space. A suitable detector is employed to probe this small volume and convert the physical and chemical properties of the passing nanoparticle labeled polymer or polymer units or monomers into an electrical signal.

In ionic current protocols, as the labeled polymer is translocated through a nanopore, any attached labels partially or totally block the current flow through the nanopore device such that each label may be detected, e.g., as a modification of current, e.g., a drop in current. In electron tunneling protocols, as the nanoparticle labeled polymer (e.g., a polymer labeled with a nanoparticle label that is conductive) moves between suitably placed tunneling electrodes associated with a nanopore, any attached nanoparticle label modifies the tunneling current that may be observed from the first electrode, across the nanoparticle labeled polymer, to the second electrode. In resonant tunneling protocols, a resonant tunneling electrode arrangement may be associated with a nanopore and the presence and energy band properties of the nanoparticle labeled polymer (e.g., a polymer labeled with a semi-conductible nanoparticle label) may be observed. Accordingly, ionic current, tunneling current and resonance tunneling current are but at least a few detector modes that are contemplated by the subject invention. In certain embodiments, the subject invention includes providing a magnetic field or circuit about a nanopore and detecting the change in the magnetic field or circuit caused by the translocation of a subject nanoparticle label (e.g., a nanoparticle label that is a magnet or has magnetic properties) through the nanopore.

By "signature" or "profile" (used herein interchangeably) is meant a collection of data points over time, in raw or processed form, e.g., in the form of a plot, such as in the form of a graphical representation, that includes a collection or series of data points obtained from a nanopore during a translocation of a nanoparticle labeled polymer versus a given time period during which an appropriate applied field or force (depending on the particular protocol performed) is applied to a nanopore and/or polymer to move the polymer into and through a nanopore. Signatures may include, but are not limited to, current profiles, magnetic field profiles, and the like. For example, by "current signature" or "current profile" (used herein interchangeably) is meant a collection of data points over time, in raw or processed form, e.g., in the form of a plot, such as in the form of a graphical representation, that includes a collection or series of current data points versus a given time period during which a polymer is translocated through a nanopore. For example, by "magnetic field signature" or "magnetic field profile" (used herein interchangeably) is meant a collection of data points over time, in raw or processed form, e.g., in the form of a plot, such as in the form of a graphical representation, that includes a collection or series of magnetic field data points versus a given time period during which a polymer is translocated through a nanopore.

Components or characteristics of the data points that may be employed include amplitude or magnitude, duration, pattern, and the like, and combinations thereof. In other words, a given data point may represent a signal that is related to the amplitude or magnitude of the detected current at given time point, etc., and the signature is a collection or series of such data points. In many embodiments two or more of these characteristics are detected when observing a nanoparticle labeled polymer translocation through a nanopore.

The given period of time that a single nanoparticle label of the subject invention is examined or detected may vary, where a given period of time may range from tens of microseconds to tens of nanoseconds, where such is at least dependant on the particular translocation rate of a given polymer through the nanopore. For example, in certain embodiments translocation rates measured may exceed about 10 x 10⁶ base-pairs/sec for double stranded DNA, where in certain embodiments the current measurement may deviate from (may be less or greater than) about 10 x 10⁶ base-pairs/sec for double stranded DNA due, for example to fluid viscosities, etc. Fluids having a viscosity near that of water may result in rates on the order of about 10 x 10⁶ base-pairs/sec.

The data points of a current signature or other analogous signature are derived from the observed or detected modulation or change in current (or magnetic field), e.g., the change in ionic, tunneling current, or resonance tunneling current through the nanopore (also referred to herein as a detector) from a first side of the nanopore, e.g., the cis side, to a second side, e.g., the *trans* side, upon occupancy of the nanopore by a nanoparticle labeled polymer. Suitable current measurement devices, as well as hardware and software necessary for generating the current signature from the detected changes in the current, including where necessary signal amplifiers and analog/digital converters, are well known in the art and thus will not be described in detail herein. Suitable magnetic field or circuit devices, as well as hardware and software necessary for generating the signature from the detected changes in the magnetic field, are also well known in the art and thus will not be described in detail herein.

### Polymer Characterization

As reviewed above, the nanoparticle labeled polymer is "read" by translocating the nanoparticle label through a nanopore and observing the effect over time of the translocation on a measurable signal. Data points in the form of a measurable signal may be obtained by observing the ion current, tunneling current, resonant tunneling current or change in magnetic field, e.g., by changing the susceptibility, through a nanopore as the subject nanoparticle labels are translocated therethrough. In this manner, a unique profile or signature such as a unique current profile or signature, magnetic field profile or signature, and the like, is generated for each nanoparticle labeled polymer, e.g., a blockade current profile and the like.

Once the current or other analogous profile is obtained by reading the nanoparticle labeled polymer as it passes through a nanopore, the resultant signature, e.g., current or other analogous profile such as magnetic field profile, may be used to provide information about the polymer such as the size of the polymer (e.g., the length of a nucleic acid sequence), analyte detection (i.e., whether a particular polymer is present or absent in a sample), identification of the polymer (e.g., identification of a nucleic acid sequence or specific nucleotide, etc.) to which the nanoparticle label was bound, whether any mutations are present, etc. These comparing and identification steps may be done manually, but are ideally performed by an appropriate computer hardware/software system. This general translocation step is shown schematically in FIGS. 6A and 6B as a nucleic acid nucleotide C, or a sequence of a nucleic acid that includes this C nucleotide, is labeled with a nanoparticle label 40. The nucleic acid of interest is passed through a suitable nanopore 111 of a nanopore device 110 under conditions suitable for a signal to be detected from the nanoparticle label as it passes through the nanopore. FIG. 6B schematically shows the signal produced from the nanopore as the labeled C nucleotide is translocated through the nanopore.

Information obtained from reading the nanoparticle labeled polymer as it passes through a nanopore may be compared against reference outputs such that the presence, absence, identity, etc., of the nanoparticle label may be determined by this comparison (or other analogous methods). Accordingly, analyte (if any) in the sample may be characterized by analyzing one or more components of the obtained signature i.e., translocation data, where components of interest include: time, amplitude, etc.

For example, reference measurements with homopolymers or heteropolymers of known composition may be made such that a decrease in ionic current observed (or a decrease or change in an analogous signal) in a given protocol may be compared to the reference to determine the identity of a polymer of interest. For example, a reference measurement (e.g., a decrease in ionic current flow) of a polymer containing stacked cytosines entering and traversing the nanopore may be made or, e.g., a reference measurement (e.g., the decrease in ionic current) of a polymer containing unstacked adenines as it enters and traverses the nanopore may be made- or any other suitable reference measurement. Accordingly, an observed decrease in current drop produced by a nanoparticle labeled polymer (e.g. DNA) of interest as it enters and traverses the nanopore may be compared to such references to determine if the polymer, e.g., DNA of interest produces the same or similar decrease in ionic current as it enters and traverses the nanopore.

### UTILITY

The subject methods find use in a variety of applications in which the detection/characterization of one or more polymers (e.g., single or double stranded nucleic acids), or sub-units of a polymer (e.g., nucleotides of a oligonucleotide) in a sample, is desired. Specific representative applications in which the subject methods find use include, but are not limited to: 1) nucleic acid size characterization applications; 2) nucleic acid analyte detection applications; 3) nucleic acid domain detection applications; and 4) nucleic acid sequencing applications. Each of these representative applications is now described in greater detail below.

### Size Characterization

As noted above, the subject invention may be employed to determine the size of a particular polymer or distinguish between different lengths of polymer in a given population of polymers. In general, nanoparticle labeled polymers may be translocated through a nanopore and the resultant signal or signature detected from the nanopore during this translocation may be related to the size of the translocated polymer. This size characterization may be accomplished, for example, by labeling two ends of a polymer with the same or different nanoparticle labels. Of course, other labeling schemes may be employed as well and will depend, e.g., on the particular polymer being characterized, etc. Because the signals provided by the nanoparticle labels are read in a sequential manner as they pass through the nanopore, the time period between detected signals provides information about the size of the polymer to which the nanoparticle labels are bound.

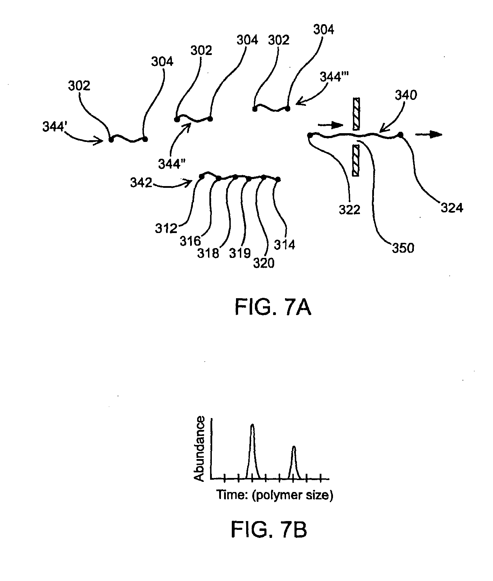

FIGS 7A and 7B illustrate an exemplary embodiment of employing the subject invention for this application. As shown in FIG. 7A, a plurality of nanoparticle labeled polymers 340, 342 and 344', 344", and 344"' of various sizes are translocated through a nanopore 350 one at a time, e.g., under an applied electric field. During translocation, detectable signals may be obtained from the nanopore due to the nanoparticle labels. As noted above, a variety of different labeling schemes may be employed in the "sizing" of a polymer. For example, only the ends of a polymer may be labeled as illustrated by polymers 340 and 344', 344", and 344"' having first and second labels 322 and 324 and 302 and 304, respectively, (represented by filled-in circles), where the first and second nanoparticle labels associated with a given polymer may be the same type or may be different types due to differences in material and/or size. As described above, in certain embodiments end-labeling DNA with a subject nanoparticle label may be accomplished by employing a 3-thiol or 5-thiol linker. Alternatively, more than just the ends of a given polymer may be labeled such as polymer 342 which has each individual residue of the polymer labeled, where the nanoparticle labels employed may be the same type or different types (e.g., some may be the same type and/or some may be different types) based on material and/or size of the nanoparticle labels.

FIG. 7B shows an output that may be obtained by employing the subject methods to size polymers. As shown, the signals obtained may be related to the time required to translocate a given polymer through a nanopore, where the translocation time is proportional to the size of the translocated polymer. Accordingly, the subject invention provides a means to easily and quickly determine the size of a polymer or a population of polymers.

### Analyte Detection

The subject invention may also be employed in analyte detection protocols. In general, such analyte detection protocols include providing a nanoparticle labeled probe (binding agent) of known identity (e.g., having a known sequence in the case of a nanoparticle labeled nucleic acid probe) and contacting the probe with a sample containing, or suspected of containing, an analyte (e.g., a complementary nucleic acid sequence) to which the nanoparticle labeled probe will bind if present. Such contact is performed under conditions sufficient to promote any such binding (e.g., under stringent hybridization conditions where the nanoparticle labeled binding agent is a nucleic acid).

Following complex formation between any nanoparticle labeled probes and analyte in the sample, in certain embodiments any unbound nanoparticle labeled probes are separated from the analyte/ nanoparticle labeled probe complexes, i.e. the bound nanoparticle labeled probes, using any suitable separation technique. Following separation of the nanoparticle labeled probe/analyte complexes from the unbound or free nanoparticle labels (if performed), as well as from any other sample constituents, the nanoparticle labeled probe/analyte complexes, (if any) may be detected and related to the presence of the analyte(s) of interest in the sample. As noted above, this detection may be accomplished by translocating the nanoparticle labeled probe/analyte complexes through a nanopore, e.g., under the influence of an applied electric field or the like and observing the effect over time of the translocation on a measurable signal, e.g., in the form of a signature. One such measurable signal is modification of ion current (or tunneling or resonant tunneling current) through a nanopore based on each unique nanoparticle label. Another such measurable signal is modification of a magnetic field (or tunneling or resonant tunneling current) through a nanopore, e.g., by changing susceptibility, based on each unique nanoparticle label. In any event, the nanoparticle labeled probe/analyte complexes are "scanned" or "read" by being translocated through a nanopore.

Once the signal output or signature, such as a current or magnetic field profile, is obtained by the translocation, the presence, absence and exact identity of the analyte to which a particular nanoparticle label probe is bound may be determined because the identity of the nanoparticle labeled probe is known. The signal obtained may be compared to reference signals to identify a particular analyte. This comparing step and identification step can be done manually, but is ideally performed by an appropriate computer hardware/software system.

Accordingly, since a signal will only be detected if a nanoparticle label is bound to an analyte and since each nanoparticle label has a unique signal and the identity of each probe is known, the identity of any probe bound analyte may be determined by relating a particular detected signal to a certain analyte. For example, the presence of a particular analyte may be determined if a particular signal is detected or alternatively if a particular signal is not detected the absence of that particular analyte may be determined. The amplitude of a particular signal may be related to the amount of a given analyte in the sample.

### Domain Detection - An Alternative to Restriction Fragment Length Polymorphis

The subject invention may be employed to easily encode a DNA molecule of interest. Specifically, the subject nanoparticle labels may be attached to one or more known sequences of a DNA molecule of interest via one or more complementary nanoparticle labeled oligonucletide probes hybridized thereto. Since the nanoparticle labels are attached to known sequences of DNA, important information about the DNA molecule may be easily obtained from the detection (or lack of detection) of one or more nanoparticle labels.

A specific use of such an approach is as an alternative to Restriction Fragment Length Polymorphism ("RFLP"). For example, regional sequence information may be obtained about the DNA molecule without employing the labor intensive method of RFLP which is conventionally used. RFLP is one way to detect base substitutions, deletions, additions and sequence re-arrangements and requires a number of time consuming and labor intensive steps. In general, RFLP requires that the DNA must first be fragmented using restriction endonucleases. The fragments are then resolved using gel electrophoresis. Differences in sequences lead to distinguishable restriction fragments, where the detection of such is employed to deduce the presence of the sequence or base of interest in the analyzed sequence.

In contrast to RFLP, using the subject invention to encode the DNA, the DNA does not have to be fragmented and the DNA may be "read" as a whole molecule to obtain analogous information as that obtainable using RFLP. For example, in using RFLP, a length of DNA of interest is cut with a restriction endonuclease. By way of example, an exemplary strand of DNA before being subject to cutting by EcoR1 may be represented as: AATCTAGGGAATTCACAGCGATGCGAATTCGCAATTA (SEQ ID NO: 02); and the same DNA after being subjected to cutting by EcoR1 may be represent as: AATCTAGGG (SEQ ID NO: 03)- AATTCACAGCGATGCG (SEQ ID NO: 04)- AATTCGCAATTA (SEQ ID NO: 05) such that the restriction endonuclease has cut the thirty-seven bases that made up the DNA strand of interest into three smaller strands of DNA having nine, sixteen and twelve bases. However, if another strand of DNA (e.g., from another person) has slightly different DNA (e.g., due to a base substitution, deletion, addition, sequence rearrangement, etc.) the same endonuclease EcoR1 may cut that particular DNA strand into pieces of different lengths. For example, in the other strand of DNA the second GAATTC may be GAATTT instead and thus the endonuclease will cut this other strand of DNA in only one place, producing only two strands of DNA of nine and twenty-eight bases each. In order to visualize these differences in DNA strands or to see if two pieces of DNA are different (i.e., whether any base substitutions, additions, deletions, sequence re-arrangements, etc.) are present, the pieces of DNA produced by a restriction endonuclease must be visualized, e.g., by performing gel electrophoresis.

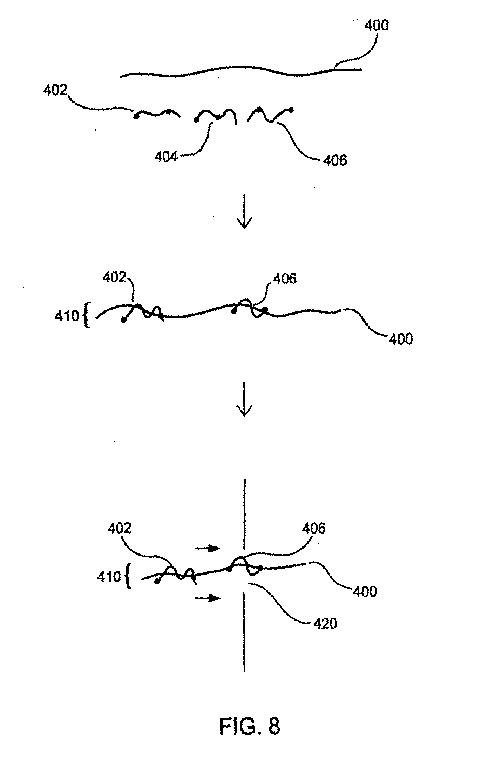

In contrast, the subject methods may be employed to provide analogous information as that which may be obtained with RFLP. As shown in FIG. 8, a DNA strand of interest 400 is provided and contacted with known sequences of nanoparticle labeled probes 402, 404 and 406 wherein the nanoparticle labels are represented by filled-in circles. The known probes may represent, e.g., polymorphisms (e.g., single nucleotide polymorphisms) or variant forms of a particular gene (e.g., may represent the complementary sequences of particular polymorphisms). The nanoparticle labeled probes are contacted with the DNA under conditions sufficient to promote the hybridization of the nanoparticle labeled probes to any complementary sequences, if any, of DNA strand 400. Accordingly, complexes formed between the probe and DNA will be indicative of the presence of a particular polymorphism (i.e., base substitution, deletion, addition, sequence re-arrangements, etc.) present within the DNA.

Following contact, any unbound nanoparticle labeled probes may be separated (or not) from the hybridized nanoparticle labeled probe/DNA complexes 410 using any suitable separation technique and any nanoparticle labeled probe/DNA complexes 410 may be detected in a manner analogous to that described above. Specifically, the complexes are detected by translocation through a nanopore 420. Translocation may be accomplished by an applied electric field, using atomic force tweezers, a magnetic force, and the like, or other suitable method of moving a polymer into and through a nanopore. Accordingly, if a nanoparticle labeled probe has hybridized to the DNA a signal may be detected from the nanopore during translocation due to the presence of the detectable nanoparticle label. The detected signal may then be related to a particular probe and thus indicative of a particular polymorphism present within DNA strand 40. Conversely, the absence of a detectable signal may be indicative of the lack of a probe and thus the absence of a polymorphism within DNA strand 40.

### Sequencing Applications

Since the subject nanoparticle labels may be discriminated from each other, the detection or lack thereof (along with the ordered sequence of detection) of the nanoparticle labels using the subject invention not only enables the determination of whether a given sequence is present or not, but also enables the order of the sequences to be easily determined. Accordingly, the subject invention may be employed to sequence all or part of a target nucleic acid molecule by determining the sequence of all or a portion of the nucleic acid molecule and/or obtaining information relating to the position of that portion of nucleic acid.

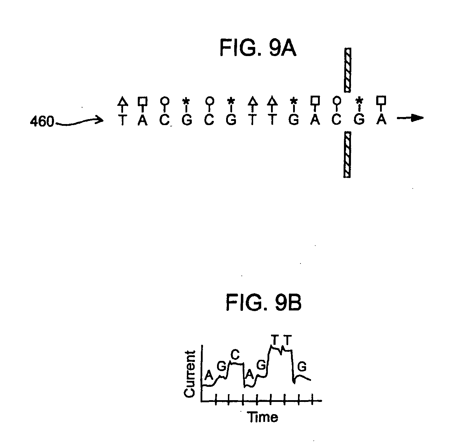

FIGS. 9A and 9B show an exemplary embodiment wherein each nucleotide of a nucleic acid 460 (SEQ ID NO:08) is labeled with a nanoparticle label of the subject invention (of course, different labeling schemes may be employed such as those that label specific sequences as opposed to individual nucleotides). Specifically, each type of nucleotide is labeled with a particular nanoparticle label. As shown, a first type of nanoparticle label is represented as a square, a second type of nanoparticle label is represented as a triangle, a third type of nanoparticle label is represented as a circle and a fourth type of nanoparticle label is represented as an asterisk. Accordingly, the sequence of the nucleic acid 460 may be determined by detecting the nucleic acid in a manner analogous to that described above. Specifically, the nanoparticle labeled nucleic acid may be translocated through a nanopore under, e.g., an applied electric or magnetic field. Particular, detectable signals may be obtained due to the presence of the nanoparticle labels. As shown in the output of such a translocation in FIG. 9B for example a translocation of eight of the nucleotides of FIG. 9A, the detected signal may be related to a particular nucleotide. Furthermore, the order in which the signals are detected provides information about the sequential order of the nucleotides. Accordingly, as shown in FIGS. 9A and 9B specific bases of a DNA of interest may be distinguishably labeled with the subject nanoparticle labels such that, as shown in FIG. 9B, different signals are provided for each base type due to different nanoparticle labels employed for each different type of base. Accordingly, current will flow in a characteristic and identifiable fashion that may be equated with a specific nucleotide (A, T, C, or G).

Specific applications in which the subject methods find use include, but are not limited to, forensics, gene mapping, diagnostic and screening applications. Diagnostic applications include applications in which the detection of one or more specific analytes in a complex physiological mixture, such as the samples described above, is desired. In such applications, the presence of the analyte(s) of interest will generally be indicative of a particular disease or condition in the host from which the screened sample is derived. Thus, in diagnostic applications according to the subject invention, the presence of one or more analytes of interest is detected in a sample from the subject being diagnosed using the methods described above and then related to the presence or absence of a disease or condition.

Other applications will be apparent to those of skill in the art. For example, the subject invention may be employed in the discovery of mutations and polymorphisms including single nucleotide polymorphisms (SNP), where these discovered mutations and polymorphisms may be in a selected portion of a gene, the full gene, the entire genome, or a subset of the genome. Such information may be valuable in the identification of mutations responsible for genetic disorders and other traits, in the identification of nucleic acid fragments, infectious agents, and the like. Still further, the subject invention may be employed in the identification of nucleic acid in parental identification. As noted above, the subject invention may be useful in forensic applications such as in the identification of nucleic acid in samples for forensic purposes.

While numerous advantages of the subject invention will be apparent to those of skill in the art upon reading this disclosure, one advantage is that the subject methods may be used to characterize polymers in a sample at very low levels. For example, in sequencing applications, the amount of polymer in a sample may be as low as about 0.05 µg/ml or lower, e.g., about 0.5 µg/ml or more, e.g., about 1 µg/ml or more in certain embodiments. For example, in detection applications, the amount of polymer in a sample may be as low as about 0.05 µg/ml or lower, e.g., about 0.5 µg/ml or more, e.g., about 1 µg/ml or more. Another advantage of the subject invention is that polymers may be characterized rapidly. For example, in sequencing applications, a polymer having a length ranging from about 50 base pairs to about 10 mega base pairs, may be sequenced rapidly, usually in less than about 30 minutes, e.g., less than about 1 minute, where the time required for sequencing may be as short as about 10 seconds or shorter, e.g., may be as short as about 1 second. In analyte detection applications, the presence of the analyte can be detected rapidly, usually in less than about 1 hour, e.g., less than about 30 minutes, where the time required for analyte detection may be as short as about 5 minutes or shorter.

### COMPUTER READABLE MEDIUMS AND PROGRAMMING STORED THEREON

One or more aspects of the subject invention may be in the form of computer readable media having programming stored thereon for implementing the subject methods. Accordingly, programming according to the subject invention may be recorded on computer-readable media, e.g., any medium that can be read and accessed directly or indirectly by a computer. Such media include, but are not limited to, computer disk or CD, a floppy disc, a magnetic "hard card", a server, magnetic tape, optical storage such as CD-ROM and DVD, electrical storage media such as RAM and ROM, and the hybrids of these categories such as magnetic/optical storage media. One of skill in the art can readily appreciate how any of the presently known computer readable mediums may be used to provide a manufacture that includes a recording of the present programming/algorithm for carrying out the above-described methodology. Thus, the computer readable media may be, for example, in the form of any of the above-described media or any other computer readable media capable of containing programming, stored electronically, magnetically, optically or by other means. As such, stored programming embodying steps for carrying-out some or all of the subject methods may be transferred to a computer-operated apparatus such as a personal computer (PC) or the like, by physical transfer of a CD, floppy disk, or like medium, or may be transferred using a computer network, server, or other interface connection, e.g., the Internet.

More specifically, a computer readable medium may include stored programming embodying an algorithm for carrying out some or all of the subject methods, where such an algorithm is used to direct a processor or series of processors to execute the steps necessary to perform the task(s) required of it and as such the subject invention includes a computer-based system for carrying-out some or all of the subject methods. For example, such a stored algorithm may be configured to, or otherwise be capable of, directing a microprocessor to receive the output signal resulting from a given translocation protocol, e.g., directly or indirectly from a signal detector, and identify the nanoparticle label and/or the polymer or polymer subunit to which the label was bound. Programming may direct a processor to provide other relevant information about a nanoparticle or a polymer or polymer subunit such as translocation time, polymer size, nucleic acid sequencing information, and the like. The algorithm may also include steps or functions for generating a variety of current profile graphs and plots. The subject invention may also include a data set of known or reference values such as current profiles or the like stored on a computer readable medium to which an output obtained from the translocation of a nanoparticle labeled polymer according to the subject invention may be compared for use in identifying a given nanoparticle and/or polymer. The data may be stored or configured in a variety of arrangements known to those of skill in the art.

### SYSTEMS

The subject invention also provides systems for use in practicing the subject methods. A system of the invention may include a suitable nanopore device, a nanoparticle labeled polymer and programming for carrying out some or all of the subject methods, which programming may be recorded on a computer readable medium as described above. In certain embodiments a subject system includes a computer or computer-based system for carrying out some or all of the methods of subject invention, e.g., for reading and implementing the algorithm.

For example, a system may include a suitable nanopore, a nanoparticle labeled polymer and a suitable single computer or suitable computer-based system or the like with a stored algorithm capable of carrying out some or all of the polymer characterization methods of the subject invention and may also include a stored data set of reference current profiles. Of course, any programming or data sets, reference values, etc., may be provided separately on a computer readable medium such that the computer or computer-based system includes means for reading the programming. In another embodiment, a system of the invention may include a suitable nanopore, a nanoparticle labeled polymer and a single computer or suitable computer-based system with a stored algorithm capable of carrying out some or all of the polymer characterization methods of the subject invention and that is configured to access a server or database that includes the data set of reference current profiles.

In certain embodiments, the system is further characterized in that it provides a user interface, where the user interface presents to a user the option of selecting amongst a plurality of different functions for evaluating a polymer according to the subject methods, for example choosing amongst one or more different, including multiple different, inputs such as voltage applied, properties of a magnetic field, the particular characteristic(s) evaluated, and the like.

### KITS

Also provided by the subject invention are kits that include the subject nanoparticle labels and instructions for using the nanoparticle labels in the subject methods. The number of different types of nanoparticle labels in the kit may range widely depending on the intended use of the kit, where the number may range from about 1 to about 16 or higher, where any nanoparticle labels are considered to be of different type if they provide different, detectable signals during translocation through a nanopore such that their signals are distinguishable from each other. Thus, kits according to the subject invention may include a single type of nanoparticle labels or two or more different types of nanoparticle labels, including sets of four or more different types of nanoparticle labels, e.g., sets of ten or more different types of nanoparticle labels, e.g., sets of sixty-four or more different types of nanoparticle labels, e.g., sets of one hundred or more different types of nanoparticle labels, e.g., sets of one thousand or more different types of nanoparticle labels, e.g., sets of one hundred thousand or more different types of nanoparticle labels and sets of five hundred thousand or more different types of nanoparticle labels. Thus, of interest are kits with at least about five, usually at least about ten , e.g., at least about fifteen different nanoparticle labels. In addition to nanoparticle labels, the kits may further include one or more additional assay components, such as suitable buffer media, and the like. The instructions for using the nanoparticle labels in the subject methods may be printed on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic, magnetic or optical storage data file present on a suitable computer readable storage medium, e.g., CD-ROM, diskette, etc.

In yet other embodiments, the instructions for using the subject nanoparticle labels in the subject methods may not themselves be present in the kit, but means for obtaining the instructions from a remote source, e.g., via the Internet, are provided. An example of this embodiment is a kit that includes a World Wide Web address where the instructions may be viewed and/or from which the instructions may be downloaded. Some form of access security or identification protocol may be used to limit access to those entitled to use the subject invention.

The subject kits may also include programming embodied on a computer readable medium as described above for carrying out one or more steps of the subject invention. The kits may also include a device for detecting and scanning or reading the nanoparticle labels, such as those described above. The kit may further include one or more additional assay components such as suitable buffer media, denaturing reagents, and the like.

### EXPERIMENTAL

The following examples are put forth so as to provide those of ordinary skill in the art with a complete disclosure and description of how to make and use the present invention, and are not intended to limit the scope of what the inventors regard as their invention. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### 1. Preparing Labeled Probes - binding gold nanoparticle labels to nucleotides

The following details how to bind gold nanoparticle labels to nucleotides. In certain embodiments the ULS, e.g., as distributed by Roche as Biotin-Chem-Link, may be employed, e.g., to bind guanine with a nanoparticle. In general, reagents for accomplishing a ULS (e.g., Biotin-Chem-Link) is incubated in aqueous solution with the nucleic acid template which leads to the cleavage of the nitrate and to the formation of a coordinative binding to the N7 position of guanosine bases.

Using purified oligonucleotides or nucleic acid templates (nucleotide bases), mix 2 µl of DNA (lug) with 1 µl of Biotin-Chem-Link. Add 18 µl of nuclease-free water and incubate for 30 minutes at 85 °C with mixing. Centrifuge briefly to collect condensate. Stop the reaction by adding 5 µl of a suitable stop solution. Store the biotin-conjugated probes at -15°C. Incubate 1 µg of biotin-conjugated probes with 1:50 strepavidin-gold (of any suitable size, e.g., 1.4 nm) and incubate for 30 minutes at room temperature. Wash three times with phosphate buffered saline ("PBS") by adding equal volumes of PBS and centrifuging briefly to remove supernatant.

In certain embodiments, bases may be labeled by providing side arms at certain positions of nucleic acid bases to which a suitable linker may be attached. Accordingly, the following protocol may be employed to modify a nucleic acid base and attach a nanoparticle via a linker such as biotin-strepavidin.

Modify one or more type of bases to provide a side arm as shown:

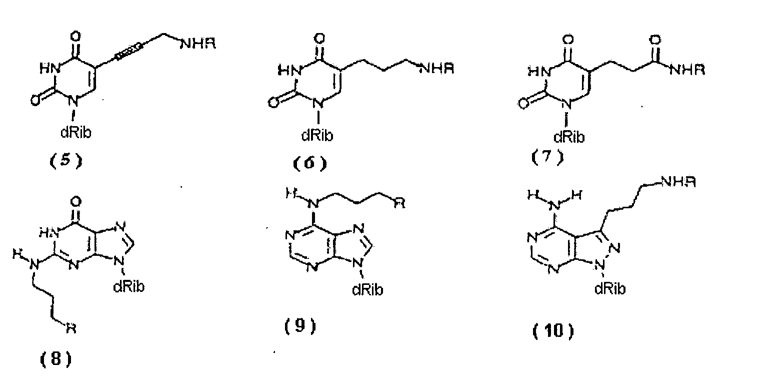

Once one or more bases have been modified to provide a suitable side arm, contact the one or more modified bases with biotin conjugate under conditions to promote the binding of the biotin conjugates to appropriate side arms. For example, one or more side arm modified bases may be incorporated into a synthesized oligonucleotide. Accordingly, following synthesis contact the synthesized oligonucleotide with biotin conjugates under conditions sufficient to promote the binding of the biotin conjugates to appropriate side arms.

Once biotin labeled, contact the biotin labeled oligonucleotides with one or more strepavidin labeled nanoparticles under conditions sufficient for biotin-strepavidin binding to occur. For example, using purified oligonucleotides or nucleic acid templates (nucleotide bases), mix 2 µl of DNA (1ug) with 1 µl of Biotin-Chem-Link. Add 18 µl of nuclease-free water and incubate for 30 minutes at 85 °C with mixing. Centrifuge briefly to collect condensate. Stop the reaction by adding 5 µl of suitable stop solution. Store the biotin-conjugated probes at -15 °C. Incubate 1 µg of biotin-conjugated probes with 1:50 strepavidin-gold (of any suitable size, e.g., 1.4 nm) and incubate for 30 minutes at room temperature. Wash three times with phosphate buffered saline ("PBS") by adding equal volumes of PBS and centrifuging briefly to remove supernatant.

### 2. Silver Enhancement of Gold Nanoparticles

The above-described protocol may also be used to bind strepavidin-gold which may be further developed to form silver around the previously produced strepavidin-gold nanoparticle label. The silver enhancement may be used, for example, to generate silver nanoparticle labels having sizes larger than the original "seed". As a result, the resultant strepavidin-silver nanoparticle labels will have a distinct electronic signature (i.e., different from the strepavidin-gold nanoparticle label seeds) due to differences in size and/or material from the "seed" strepavidin-gold nanoparticle label. The strepavidin-silver nanoparticle labels may range in size from about 2 to about 10 nm. Materials for such silver enhancement may be found commercially, e.g., from Nanoprobes.

### 3. Linker-Conjugated Nanoparticle Labels

As described above, nanoparticle labels may be conjugated to different linkers, e.g., for the incorporation of the nanoparticle label into dNTP's. For example, using silanization, covalently couple carboxy functional groups to the surface of a gold nanoparticle. Activate the carboxy functionalized surface with carbodiimide. Once activated, contact the nanoparticle with strepavidin under conditions sufficient to bind the strepavidin to the activated nanoparticle surface. Alternatively, a thiol linker may be employed. For example, to provide a thiol linked DNA molecule to a nanoparticle, modify a nanoparticle such as a gold nanoparticle by contacting it with 3' or 5' thiol functionalized DNA having a length at least about 25 bases, under conditions sufficient to stably attach the 3' or 5' thiol functionalized DNA to the nanoparticle to provide a DNA linker. Still further, modification of nanoparticles may be accomplished by employing ligand exchange reactions of phosphine-stabilized nanoparticles with ω-functionalized alkanethiols to modify gold nanoparticles with ω-functionalized alkanethiols.

### 4. Encoded DNA

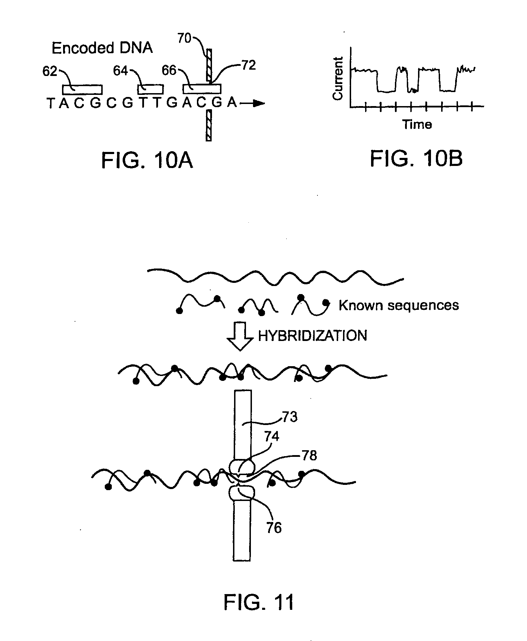

The subject nanoparticle labels may be used to encode specific sequences of DNA which then enables the identification of particular segments of the DNA of interest. FIGS. 10A and 10B illustrate this protocol. The encoding may take the form of various nanoparticle labels associated with a probe, e.g., the nanoparticle labels may be positioned at opposing ends of a probe of known sequence or the like.

Accordingly, suitably label one or more probe molecules of known sequence with one or more nanoparticle labels of the subject invention, e.g., 1.4 nm and 0.8 nm gold and/or silver nanoparticle labels. Contact with a sample containing or suspected of containing DNA of interest under conditions sufficient to promote the hybridization of the nanoparticle labeled probe(s) to the DNA of interest which has been denatured into single stranded DNA. Following the hybridization of the nanoparticle labeled probe(s) to the DNA of interest, translocate the hybridized complex through a nanopore by applying a DC current of about 120 mV across the nanopore in the presence of an electrolyte buffer such as 1m KCL in 10 mM Tris-Cl pH 8.5 for a period of time sufficient to translocate the hybridized complex through (or partially through in certain embodiments) the nanopore, e.g., for a period of time that ranges from about 10 minutes to about several hours.

As shown in FIG. 10A, a plurality of nanoparticle labeled probes 62, 64 and 66 are hybridized to the DNA of interest and are translocated through pore 72 of nanopore device 70 by applying a voltage across the pore in the presence of an electrolyte buffer. As shown in FIG. 10B, the signal from this translocation is observed as a blockage of ionic current indicating the DNA and label are blocking the nanopore resulting in a decrease in ionic current. The degree or magnitude of blocking is due in part to the properties (i.e., the sizes) of the subject nanoparticle labels employed and thus each different nanoparticle label imparts a unique blocking signal. The graph of FIG. 10B shows exemplary time and amplitude components of the translocation which is related to the length and type of DNA being translocated. For example, longer lengths of DNA will have longer translocation times and the amplitude of blockage may be related to the conformation of the DNA and/or to the labeled state of the DNA. Furthermore, since the subject nanoparticle labels are hybridized to known sequences of DNA, sequencing information may be obtained about the DNA of interest using an intact DNA molecule (as opposed to fragmenting the DNA using restriction endonucleases) and without the need to employ conventional protocols that may be more labor-intensive such as restriction length polymorphism ("RLP").

### 5. Tunneling Current and Resonant Tunneling Current Examples

Characterization of a polymer (DNA, RNA, protein, etc.) may be accomplished using the subject nanoparticle labels in tunneling current protocols and resonant tunneling current protocols as shown schematically in FIG. 11. Accordingly, nanoparticle labels having different conductivities may be employed as labels for a given polymer to enable discrimination of the different nanoparticle labels and thus discrimination of the respective unit or sequence, etc., of the polymer to which a given label is associated, e.g., nanoparticle labels of different materials and/or sizes may be employed.

Analogous to that described above, label probes of known sequence (labels represented by filled-in circles) with nanoparticle labels that are conductive or semiconductive. Once labeled, hybridize the labeled probes to DNA of interest. The hybridized, labeled complex is passed through two tunneling current electrodes 74 and 76 positioned at the periphery of the nanopore 78 of nanopore device 73. During movement of the hybridized, labeled complex through the nanopore, the hybridized, labeled complex provides a signal output due to the conductive/ semiconductive properties of the nanoparticle labels.

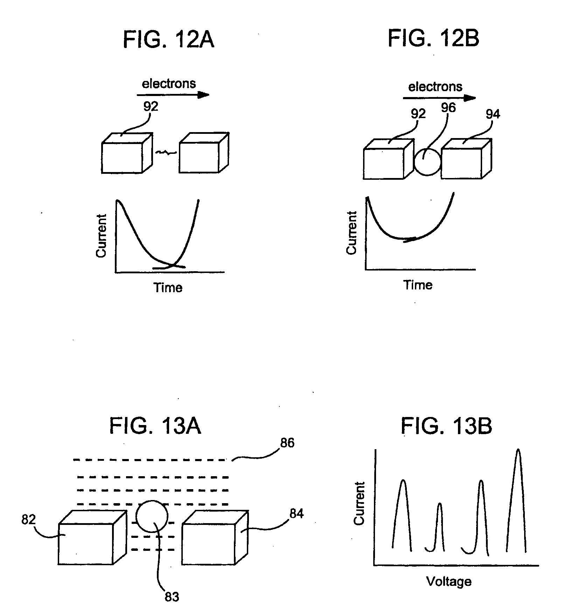

In the case of tunneling current, the use of conductive or semiconductive nanoparticle labels increases the likelihood that electrons will conduct across the electrodes, as shown in FIGS. 12A and 12B. As shown in FIG. 12A, the magnitude of the current conducting across tunneling electrodes 92 and 94 is relatively low as compared to the current conducting across electrodes 92 and 94 when nanoparticle labeled DNA 96, generally represented as circle 96, is moved through the nanopore, as shown in FIG. 12B.

In the case of resonant tunneling current, the nanoparticle labeled DNA traverses a plurality of energy barriers or levels as it moves through the nanopore where such movement across the various energy barriers produces a spectrum of current that is a unique signature for a given nanoparticle label. In this case, the nanoparticle may be conductive or semiconductive, e.g., a quantum dot. This is schematically illustrated in FIGS 13A and 13B. FIG. 13A shown resonant tunneling electrodes 82 and 84, energy bands 86 generally represented as dashed lines and nanoparticle labeled DNA 83, generally represented as circle 83, moving through the various energy bands 86. FIG. 13B shows an exemplary signal output produced from such a resonant tunneling current protocol such that a spectrum of currents is detected as the nanoparticle labeled DNA passes through the various energy levels.

### 6. Magnetic Detection Circuit for Detecting Magnetic Nanoparticle Labels

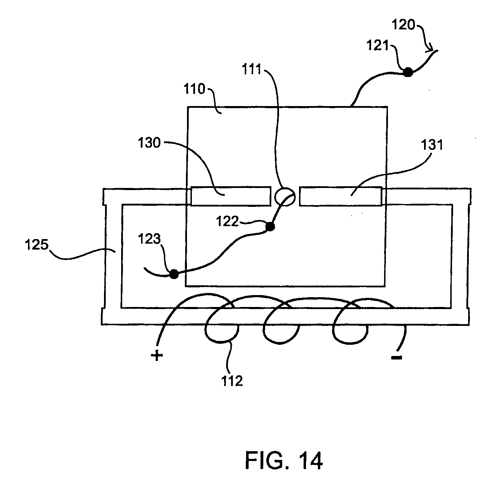

Characterization of a polymer (DNA, RNA, protein, etc.) may be accomplished using one or more of the magnetic nanoparticle labels of the subject invention associated with the polymer and detecting signal from a magnetic circuit associated-nanopore during translocation of the magnetic particle-labeled polymer through the nanopore, e.g., by sensing magnetic susceptibility or rather a change thereof, or the like, as shown schematically in FIG. 14 which shows an end view of a nanopore device having a nanopore associated with a magnetic circuit. In certain embodiments a plurality of magnetic nanoparticle labels that provide different signals are employed as labels for a given polymer to enable discrimination of the different magnetic nanoparticle labels and thus discrimination of the respective unit or sequence, etc., of the polymer to which a given label is associated, e.g., magnetic nanoparticle labels of different materials and/or sizes may be employed.

As shown in FIG. 14, polymer 120 labeled with magnetic nanoparticle labels 121, 122 and 123 (which may be the same type of magnetic nanoparticle labels or may be different) is passed through nanopore 111 of nanopore device 110 (labeled polymer 120 is depicted as moving through the nanopore in the direction that corresponds to movement of the polymer out of the page). The magnetic circuit 125 includes coil 112 that is magnetically coupled (via ferrite or other suitable magnetic material) to stripes of magnetic film 130 and 131 which in turn localize the field in the proximity of nanopore 111, thereby enabling the magnetic nanoparticle labels associated with the polymer to affect the field by changing the susceptibility. Accordingly, contact the labeled polymer with the nanopore to cause the traversal of the magnetic nanoparticle labeled polymer through the nanopore. Detect a signal which is indicative of this change in magnetic susceptibility caused by the traversed nanoparticle label and characterize the polymer based on this detected signal.

It is evident from the above results and discussion that the above described invention provides effective methods and devices for characterizing a polymer. The subject invention provides for a number of advantages including, but not limited to, unique labels that are easy to use and which provide unique signals. Furthermore, in certain embodiments the nanoparticle labels may be read rapidly in much shorter times than that required for methods in which other labels, such as radioactive or fluorescent labels are employed. As such, the subject invention represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

While the present invention has been described with reference to the specific embodiments thereof, it should be understood by those skilled in the art that various changes may be made and equivalents may be substituted without departing from the scope of the invention. In addition, many modifications may be made to adapt a particular situation, material, composition of matter, process, process step or steps, to the objective, and scope of the present invention. All such modifications are intended to be within the scope of the claims appended hereto.

## Claims

1. A method of characterizing a polymer (120, 340, 342, 344, 400, 460) in a sample, said method comprising:
(a) contacting said sample comprising said polymer (120, 340, 342, 344, 400, 460) with a nanopore (111, 350, 420) under conditions so that said polymer (120, 340, 342, 344, 400, 460) translocates through said nanopore (111, 350, 420), wherein said polymer (120, 340, 342, 344, 400, 460) is labeled with at least one nanoparticle label (20, 21, 27, 28, 29, 40, 121, 122, 123); and
(b) reading a signal from said nanopore (111, 350, 420) to characterize said polymer (120, 340, 342, 344, 400, 460) in said sample.

2. A method as claimed in Claim 1, wherein said at least one nanoparticle label (20, 21, 27, 28, 29, 40, 121, 122, 123) is chosen from gold, silver, copper, tin, titanium, iron, cobalt, aluminum, zinc, bismuth, zirconia, cerium, magnesium, copper oxide, tin oxide, titanium dioxide, indium tin oxide, antimony tin oxide, barium titanate and calcium oxide.

3. A method as claimed in Claim 1 or 2, wherein said at least one nanoparticle label (20, 21, 27, 28, 29, 40, 121, 122, 123) has a diameter that ranges from about 0.5 nm to about 35 nm.

4. A method as claimed in any of Claims 1, 2 or 3, wherein said polymer (120, 340, 342, 344, 400, 460) is labeled with a plurality of said nanoparticle labels (20, 21, 27, 28, 29, 40, 121, 122, 123).

5. A method as claimed in Claim 4, wherein all of said nanoparticle labels (20, 21, 27, 28, 29, 40, 121, 122, 123) produce the same detectable signal.

6. A method as claimed in Claim 4, wherein two or more of said nanoparticle labels (20, 21, 27, 28, 29, 40, 121, 122, 123) produce different detectable signals.

7. A method as claimed in any preceding Claim, wherein said polymer (120, 340, 342, 344, 400, 460) is a nucleic acid that comprises at least two different types of sub-units and each different sub-unit is labeled with a different type ofnanoparticle label (20, 21, 27, 28, 29, 40, 121, 122, 123).

8. A method as claimed in any preceding Claim, wherein said at least one nanoparticle label (20, 21, 27, 28, 29, 40, 121, 122, 123) is a conductive nanoparticle label.

9. A method as claimed in any preceding Claim, wherein said at least one nanoparticle label (20, 21, 27, 28, 29, 40, 121, 122, 123) is a semiconductive nanoparticle label.

10. A method as claimed in any preceding Claim, wherein said at least one nanoparticle label (20, 21, 27, 28, 29, 40, 121, 122, 123) is a magnetic nanoparticle label.

11. A method as claimed in any preceding Claim, wherein said reading step comprises observing the tunneling current effect of said translocation.

12. A method as claimed in any preceding Claim, wherein said reading step comprises observing the resonance tunneling current effect of said translocation.

13. A method as claimed in any preceding Claim, wherein said reading step comprises observing the ionic current effect of said translocation.

14. A method as claimed in any preceding Claim, wherein said reading step comprises observing the change in magnetic susceptibility.

15. A computer-readable medium comprising programming for a method of characterizing a polymer as claimed in any preceding Claim.
